# FASCICULE DE BREVET EUROPEEN

(11) **EP 2 198 841 B1**
(45) Date de publication et mention de la délivrance du brevet: **23.01.2019**
(21) Numéro de dépôt: 09179888.4
(22) Date de dépôt: 18.12.2009
(51) Int. Cl.: A61K 8/31, A61K 8/41, A61K 8/86, A61Q 5/08, A61K 8/37, A61K 8/06

(54) **Procédé d'éclaircissement de matières kératiniques mettant en oeuvre une émulsion comprenant un agent alcalin et une composition oxydante**
Verfahren zur Aufhellung keratinischer Fasern mit einer Emulsion enthaltend eine alkalische Komponente und einer oxidierenden Zubereitung
Method for lightening keratinic fibers with an emulsion containing an alkaline agent and an oxidising composition.

(30) Priorité: 19.12.2008 FR 0858890
(43) Date de publication de la demande: 23.06.2010
(73) Titulaire: L'Oréal, 75008 Paris (FR)
(72) Inventeur: Hercouet, Leïla, 93360, Neuilly Plaisance (FR); Bernard, Anne-Laure, 92160, Antony (FR); Bordeaux, Dominique, 91450, Soisy sur Seine (FR)
(74) Mandataire: Fevrier, Murielle Françoise E.

(56) Documents cités:
- EP-A- 1 813 254
- CA-A1- 2 573 567
- DE-A1- 3 814 356
- FR-A- 2 910 309
- JP-A- 2003 055 174
- US-A- 5 817 155
- US-A1- 2003 190 297
- US-A1- 2006 242 773

## Description

La présente invention a pour objet un procédé d'éclaircissement des matières kératiniques humaines, notamment les cheveux.

Les procédés d'éclaircissement des matières kératiniques telles que les fibres kératiniques humaines consistent à employer une composition aqueuse comprenant au moins un agent oxydant, en condition de pH alcalin dans la grande majorité des cas. Cet agent oxydant a pour rôle de dégrader la mélanine des cheveux, ce qui, en fonction de la nature de l'agent oxydant présent, conduit à un éclaircissement plus ou moins prononcé des fibres. Ainsi, pour un éclaircissement relativement faible, l'agent oxydant est généralement le peroxyde d'hydrogène. Lorsqu'un éclaircissement plus important est recherché, on met habituellement en oeuvre des sels peroxygénés, comme des persulfates par exemple, en présence de peroxyde d'hydrogène.

L'une des difficultés vient du fait que le procédé d'éclaircissement est mis en oeuvre dans des conditions alcalines et que l'agent alcalin le plus couramment utilisé est l'ammoniaque. L'ammoniaque est particulièrement avantageux dans ce type de procédé. En effet, il permet d'ajuster le pH de la composition à un pH alcalin pour permettre l'activation de l'agent oxydant. Cet agent provoque également un gonflement de la fibre kératinique, avec une ouverture des écailles, ce qui favorise la pénétration de l'oxydant à l'intérieur de la fibre et donc augmente l'efficacité de la réaction.

Or cet agent alcalinisant est très volatil, ce qui occasionne des désagréments à l'utilisateur du fait de l'odeur caractéristique forte, plutôt incommodante de l'ammoniac qui se dégage durant le procédé.

De plus, la quantité d'ammoniac dégagée nécessite l'emploi de teneurs plus importantes que nécessaires pour compenser cette perte. Cela n'est pas sans conséquence pour l'utilisateur qui reste non seulement incommodé par l'odeur mais qui peut également être confronté à des risques plus importants d'intolérance, comme par exemple une irritation du cuir chevelu (picotements).

Quant à l'option de purement et simplement remplacer en totalité ou en partie l'ammoniaque par un ou plusieurs autres agents alcalinisants classiques, celle-ci ne conduit pas à des compositions aussi efficaces que celles à base d'ammoniaque, notamment parce que ces agents alcalinisants ne conduisent pas un éclaircissement suffisant des fibres pigmentées en présence de l'agent oxydant.

L'un des objectifs de la présente invention est de proposer des procédés d'éclaircissement des matières kératiniques, notamment des fibres kératiniques telles que les cheveux qui ne présentent pas les inconvénients de ceux mis en oeuvre avec les compositions existantes, inconvénients causés par la présence de teneurs importantes en ammoniaque, tout en restant au moins aussi efficaces sur le plan de l'éclaircissement et de l'homogénéité de ce dernier.

Ces buts et d'autres sont atteints par la présente invention qui a donc pour objet un procédé d'éclaircissement de matières kératiniques dans lequel on met en oeuvre :
a) une émulsion directe (A) comprenant un ou plusieurs corps gras tel que défini dans la revendication 1 en quantité supérieure à 25 % en poids, de préférence plus de 50 % ; un ou plusieurs tensioactifs ; un ou plusieurs agents alcalins et une quantité d'eau supérieure à 5 % en poids, du poids total de l'émulsion,
b) une composition aqueuse (B) comprenant un ou plusieurs agents oxydants.

Elle concerne également un dispositif à plusieurs compartiments comprenant dans l'un d'eux une émulsion (A), dans un autre une composition (B) comprenant un ou plusieurs agents oxydants.

Dans le cadre de l'invention, une émulsion directe est une émulsion huile dans eau.

Dans ce qui va suivre, et à moins d'une autre indication, les bornes d'un domaine de valeurs sont comprises dans ce domaine.

Les matières kératiniques traitées par le procédé selon l'invention sont par exemple, les poils, les cils et les cheveux. Le procédé de l'invention permet notamment d'obtenir un bon niveau d'éclaircissement de ces matières kératiniques telles que les cheveux sans dégagement d'une odeur d'ammoniaque, pouvant être irritante.

L'émulsion (A) présente plus particulièrement une teneur en eau inférieure à 50 % en poids, de préférence comprise entre 10 et 50 % en poids, par rapport au poids de l'émulsion.

En outre, selon un mode de réalisation particulier, l'émulsion directe (A) ne comprend pas de colorant direct ou de précurseur de colorant d'oxydation (bases et coupleurs) utilisés habituellement pour la coloration des fibres kératiniques humaines ou bien, si elle en comprend, leur teneur totale ne dépasse pas 0,005 % en poids par rapport au poids de l'émulsion eau dans huile. En effet, à une telle teneur, seule l'émulsion serait teintée, c'est-à-dire qu'on n'observerait pas d'effet de coloration des fibres kératiniques.

L'émulsion huile dans eau utile dans la présente invention comprend un ou plusieurs corps gras.

Par corps gras, on entend, un composé organique insoluble dans l'eau à température ordinaire (25°C) et à pression atmosphérique (760 mm de Hg) (solubilité inférieure à 5% et de préférence à 1% encore plus préférentiellement à 0,1%). Ils présentent dans leur structure un enchaînement d'au moins deux groupements siloxane ou au moins une chaine hydrocarbonée comportant au moins 6 atomes de carbone. En outre, les corps gras sont généralement solubles dans les solvants organiques dans les mêmes conditions de température et de pression, comme par exemple le chloroforme, l'éthanol, le benzène ou le décaméthylcyclopentasiloxane.

Selon la présente invention, le ou les corps gras sont différents des acides gras.

Les corps gras sont notamment choisis parmi les alcanes inférieurs, les alcools gras, les esters d'acide gras, les esters d'alcool gras et les huiles en particulier les huiles non siliconées minérales, végétales, animales ou synthétiques. comprennent de préférence de 6 à 16 atomes de carbone et sont linéaires ou ramifiés, éventuellement cycliques. A titre d'exemple, les alcanes peuvent être choisis parmi l'hexane et le dodécane, les isoparaffines comme l'isohexadécane et l'isodécane.

Comme huiles non siliconées utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité ;
- les hydrocarbures de plus de 16 atomes de carbone linéaires ou ramifiés, d'origine minérale ou synthétique, tels que les huiles de paraffine, volatiles ou non, et leurs dérivés, la vaseline, l'huile de vaseline, les polydécènes, le polyisobutène hydrogéné tel que Parléam®.
- les huiles fluorées partiellement hydrocarbonées ; comme huiles fluorées, on peut citer aussi le perfluorométhylcyclopentane et le perfluoro-1,3 diméthylcyclohexane, vendus sous les dénominations de "FLUTEC® PC1" et "FLUTEC® PC3" par la Société BNFL Fluorochemicals; le perfluoro-1,2-diméthylcyclobutane; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M.

Les alcools gras utilisables comme corps gras dans la composition de l'invention sont non oxyalkylénés, saturés ou insaturés, linéaires ou ramifiés, et comportent 6 à 30 atomes de carbone et plus particulièrement de 8 à 30 atomes de carbone, on peut citer l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

Les esters sont les esters de mono ou polyacides aliphatiques saturés ou insaturés, linéaires ou ramifiés en C1-C26 et de mono ou polyalcools aliphatiques saturés ou insaturés, linéaires ou ramifiés en C1-C26, le nombre total de carbone des esters étant plus particulièrement supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en C12-C15 ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le malate de dioctyle, le laurate d'hexyle, le laurate de 2-hexyldécyle.

Toujours dans le cadre de cette variante, on peut également utiliser les esters d'acides di ou tricarboxyliques en C4-C22 et d'alcools en C1-C22 et les esters d'acides mono di ou tricarboxyliques et d'alcools di, tri, tétra ou pentahydroxy en C2-C26.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition peut également comprendre, à titre d'ester gras, des esters et di-esters de sucres d'acides gras en C6-C30, de préférence en C12-C22. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C6-C30, de préférence en C12-C22, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou di- oléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

Les silicones utilisables dans la composition de la présente invention, sont des silicones volatiles ou non volatiles, cycliques, linéaires ou ramifiées, modifiées ou non par des groupements organiques, ayant une viscosité de 5.10-6 à 2,5m2/s à 25°C et de préférence 1.10-5 à 1m2/s.

De préférence, les corps gras ne sont ni oxyalkylénés, ni glycérolés.

Plus particulièrement, les corps gras sont choisis parmi les composés liquides ou pâteux à température ambiante et à pression atmosphérique.

De préférence, le corps gras est un composé liquide à la température de 25°C et à la pression atmosphérique.

Selon un mode de réalisation particulier le ou les corps gras liquides ont un poids moléculaire supérieur ou égal à 360 g/mol.

Les corps gras sont de préférence choisis parmi les alcanes inférieurs, les alcools gras, les esters d'acide gras, les esters d'alcool gras, les huiles en particulier les huiles non siliconées minérales, végétales ou synthétiques, les silicones

Selon un mode de réalisation, le ou les corps gras est ou sont choisis parmi l'huile de vaseline, les polydécènes, les esters d'acides gras ou d'alcools gras, liquides ou leurs mélanges, en particulier, le ou les corps gras de la composition selon l'invention sont non siliconés.

On choisira de préférence les alcanes ou hydrocarbures et les silicones.

La composition selon l'invention comprend au moins 25 % de corps gras. De préférence la concentration en corps gras va de 25 à 80 %, encore plus préférentiellement de 25 à 65 %, mieux de 30 à 55 % du poids total de la composition.

L'émulsion (A) comprend également un ou plusieurs tensioactifs.

De préférence, le ou les tensioactifs sont choisis parmi les tensioactifs non ioniques ou parmi les tensioactifs anioniques, de préférence les tensioactifs non ioniques.

Les tensioactifs anioniques sont par exemple choisis parmi les sels (en particulier sels de métaux alcalins, notamment de sodium, sels d'ammonium, sels d'amines, sels d'aminoalcools ou sels de métaux alcalino-terreux comme le magnésium) des composés suivants :
- les alkylsulfates, les alkyléthersulfates, alkylamidoéthersulfates, alkylaryl-polyéthersulfates, monoglycérides sulfates ;
- les alkylsulfonates, alkylamidesulfonates, alkylarylsulfonates, α-oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Il est à noter que le radical alkyle ou acyle de ces différents composés comporte avantageusement de 6 à 24 atomes de carbone, et de préférence de 8 à 22 atomes de carbone et encore plus préférentiellement de 18 à 22 atomes de carbone, , et le radical aryle désignant de préférence un groupement phényle ou benzyle

Les tensioactifs non ioniques sont plus particulièrement choisis parmi les tensioactifs non ioniques mono ou poly- oxyalkylénés, mono- ou poly- glycérolés. Les motifs oxyalkylénés sont plus particulièrement des motifs oxyéthylénés, oxypropylénés, ou leur combinaison, de préférence oxyéthylénés.

A titre d'exemples de tensioactifs non ioniques oxyalkylénés, on peut citer :
- les alkyl(C₈-C₂₄)phénols oxyalkylénés,
- les alcools en C₈-C₃₀, saturés ou non, linéaires ou ramifiés, oxyalkylénés,
- les amides, en C₈-C₃₀, saturés ou non, linéaires ou ramifiés, oxyalkylénés,
- les esters d'acides en C₈-C₃₀, saturés ou non, linéaires ou ramifiés, et de polyéthylèneglycols,
- les esters d'acides en C₈-C₃₀, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres, seuls ou en mélanges.

Les tensioactifs présentant un nombre de moles d'oxyde d'éthylène et/ou de propylène compris entre 1 et 50, de préférence entre 2 et 30. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en C₈-C₃₀ oxyéthylénés., de préférence C₁₈-C₃₀ oxyéthylénés.

Comme alcools gras éthoxylés, on peut citer par exemple les produits d'addition d'oxyde d'éthylène avec l'alcool laurylique, notamment ceux comportant de 9 à 50 groupes oxyéthylénés et plus particulièrement ceux comportant de 10 à 12 groupes oxyéthylénés (Laureth-10 à Laureth-12 en noms CTFA) ; les produits d'addition d'oxyde d'éthylène avec l'alcool béhénylique, notamment ceux comportant de 9 à 50 groupes oxyéthylénés (Beheneth-9 à Beheneth-50 en noms CTFA) de préférence 10 groupes oxyéthylénés. (Beheneth-10); les produits d'addition d'oxyde d'éthylène avec l'alcool cétéarylique (mélange d'alcool cétylique et d'alcool stéarylique), notamment ceux comportant de 10 à 30 groupes oxyéthylénés (Ceteareth-10 à Ceteareth-30 en noms CTFA) ; les produits d'addition d'oxyde d'éthylène avec l'alcool cétylique, notamment ceux comportant de 10 à 30 groupes oxyéthylénés (Ceteth-10 à Ceteth-30 en noms CTFA) ; les produits d'addition d'oxyde d'éthylène avec l'alcool stéarylique, notamment ceux comportant de 10 à 30 groupes oxyéthylénés (Steareth-10 à Steareth-30 en noms CTFA) ; les produits d'addition d'oxyde d'éthylène avec l'alcool isostéarylique, notamment ceux comportant de 10 à 50 groupes oxyéthylénés (Isosteareth-10 à Isosteareth-50 en noms CTFA) ; et leurs mélanges.

Comme acides gras éthoxylés, on peut citer par exemple les produits d'addition d'oxyde d'éthylène avec les acides laurique, palmitique, stéarique ou béhénique, et leurs mélanges, notamment ceux comportant de 9 à 50 groupes oxyéthylénés tels que les laurates de PEG-9 à PEG-50 (en noms CTFA : PEG-9 laurate à PEG-50 laurate) ; les palmitates de PEG-9 à PEG-50 (en noms CTFA : PEG-9 palmitate à PEG-50 palmitate) ; les stéarates de PEG-9 à PEG-50 (en noms CTFA : PEG-9 stearate à PEG-50 stearate) ; les palmito-stéarates de PEG-9 à PEG-50 ; les béhénates de PEG-9 à PEG-50 (en noms CTFA : PEG-9 behenate à PEG-50 behenate) ; et leurs mélanges.

Conformément à un mode de réalisation préféré de l'invention, les tensioactifs non ioniques oxyalkylénés sont choisis parmi les alcools en C₁₈-C₃₀, oxyéthylénés.

On peut utiliser aussi des mélanges de ces dérivés oxyéthylénés d'alcools gras et d'acides gras.

Selon un mode préféré de réalisation, l'émulsion (A) comprend au moins un alcool gras éthoxylé, et de préférence au moins l'alcool béhénylique.

A titre d'exemple de tensioactifs non ioniques mono- ou poly- glycérolés, on utilise de préférence les alcools en C₈-C₄₀, mono- ou poly- glycérolés.

En particulier, les alcools en C₈-C₄₀ mono- ou poly- glycérolés correspondent à la formule suivante :

RO-[CH₂-CH(CH₂OH)-O]ₘ-H

dans laquelle R représente un radical alkyle ou alcényle, linéaire ou ramifié, en C₈-C₄₀, de préférence en C₈-C₃₀, et m représente un nombre allant de 1 à 30 et de préférence de 1 à 10.

A titre d'exemple de composés convenables dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en C₈/C₁₀ à une mole de glycérol, l'alcool en C₁₀/C₁₂ à 1 mole de glycérol et l'alcool en C₁₂ à 1,5 mole de glycérol.

De préférence, le tensioactif présent dans l'émulsion est un tensioactif non ionique présentant un HLB de 8 à 18. Le HLB est le rapport entre la partie hydrophile et la partie lipophile dans leur molécule. Ce terme HLB est bien connu de l'homme du métier et est décrit dans "The HLB system. A time-saving guide to Emulsifier Selection" (published by ICI Americas Inc ; 1984).

La teneur en tensioactifs dans l'émulsion (A) représente plus particulièrement de 0,1 à 50 % en poids, de préférence de 0,5 à 30 % en poids par rapport au poids de la composition anhydre.

L'émulsion utile dans la présente invention comprend un ou plusieurs agents alcalins.

L'agent alcalin peut être choisi parmi les bases minérales, les amines organiques, les sels d'amines organiques, seuls ou en mélange.

A titre d'exemple d'amine organique, on peut citer les amines organiques dont le pKb à 25°C est inférieur à 12, et de préférence inférieur à 10, encore plus avantageusement inférieur à 6. Il est à noter qu'il s'agit du pKb correspondant à la fonction de basicité la plus élevée.

L'amine organique peut comprendre une ou deux fonctions amine primaire, secondaire ou tertiaire, et un ou plusieurs groupements alkyle, linéaires ou ramifiés, en C₁-C₈ porteurs d'un ou plusieurs radicaux hydroxyle.

Conviennent en particulier à la réalisation de l'invention les amines organiques choisies parmi les alcanolamines telles que les mono-, di- ou tri- alcanolamines, comprenant un à trois radicaux hydroxyalkyle, identiques ou non, en C₁-C₄.

Parmi des composés de ce type, on peut citer la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylaminométhane.

Conviennent également les amines organiques de formule suivante : 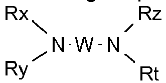 dans laquelle W est un reste alkylène en C₁-C₆ éventuellement substitué par un groupement hydroxyle ou un radical alkyle en C₁-C₆ ; Rx, Ry, Rz et Rt, identiques ou différents, représentent un atome d'hydrogène, un radical alkyle en C₁-C₆ ou hydroxyalkyle en C₁-C₆, aminoalkyle en C₁-C₆.

On peut citer à titre d'exemple de telles aminés, le 1,3 diaminopropane, le 1,3 diamino 2 propanol, la spermine, la spermidine.

Selon une autre variante de l'invention, l'amine organique est choisie parmi les acides aminés.

Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.

De manière avantageuse, les acides aminés sont des acides aminés basiques comprenant une fonction amine supplémentaire éventuellement incluse dans un cycle ou dans une fonction uréido.

De tels acides aminés basiques sont choisis de préférence parmi ceux répondant à la formule (I) suivante : 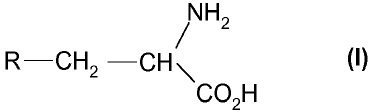 où R désigne un groupe choisi parmi : 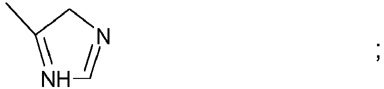 -(CH₂)₃NH₂ ; -(CH₂)₂NH₂ ; -(CH₂)₂NHCONH₂ ;

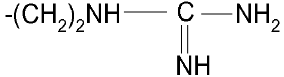

Les composés correspondants à la formule (I) sont l'histidine, la lysine, l'arginine, l'ornithine, la citrulline.

A titre d'acides aminés utilisables dans la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

Selon une variante préférée de l'invention, l'amine organique est choisie parmi les acides aminés basiques. Les acides aminés particulièrement préférés sont l'arginine, la lysine, l'histidine, ou leurs mélanges.

Selon une autre variante de l'invention, l'amine organique est choisie parmi les amines organiques de type hétérocycliques On peut en particulier citer, outre l'histidine déjà mentionnée dans les acides aminés, la pyridine, la pipéridine, l'imidazole, le 1,2,4-triazole, le tétrazole, le benzimidazole.

Selon une autre variante de l'invention, l'amine organique est choisie parmi les dipeptides d'acides aminés. A titre de dipeptides d'acides aminés utilisables dans la présente invention, on peut notamment citer la carnosine, l'anserine et la baleine

Selon une autre variante de l'invention, l'amine organique est choisie parmi les composés comportant une fonction guanidine. A titre d'amines d'amines de ce type utilisables dans la présente invention, on peut notamment citer outre l'arginine déjà mentionnée à titre d'acide aminé, la créatine, la créatinine, la 1,1-diméthylguanidine, 1,1-diéthylguanidine, la glycocyamine, la metformin, l'agmatine, la n-amidinoalanine, l'acide 3-guanidinopropionique, l'acide 4-guanidinobutyrique et l'acide 2-([amino(imino)methyl]amino)ethane-1-sulfonique.

De préférence l'amine organique est une alcanolamine. Plus préférentiellement l'amine organique est choisie parmi le 2-amino 2-méthyl 1-propanol, la monoéthanolamine ou leurs mélanges. Encore plus préférentiellement l'amine organique est la monoéthanolamine.

L'agent alcalin peut être une amine organique sous forme de sels. Par sel d'amine organique, on entend au sens de la présente invention, les sels organiques ou inorganiques d'une amine organique telle que décrite ci-dessus.

De préférence, les sels organiques sont choisis parmi les sels d'acides organiques tels que les citrates, les lactates, les glycolates, les gluconates, les acétates, les propionates, les fumarates, les oxalates et les tartrates.

De préférence, les sels inorganiques sont choisis parmi les halogénohydrates (chlorhydrates par exemple), les carbonates, les hydrogénocarbonates, les sulfates, les hydrogénophosphates et les phosphates.

Par base inorganique, au sens de la présente invention, on entend tout composé possédant dans sa structure un ou plusieurs éléments des colonnes 1 à 13 du tableau périodique des éléments autres que l'hydrogène, ne comportant pas simultanément d'atome(s) de carbone et d'hydrogène.

Selon un mode de réalisation particulier de l'invention, la base inorganique contient un ou plusieurs éléments des colonnes 1 et 2 du tableau périodique des éléments autres que l'hydrogène.

Dans une variante préférée la base inorganique présente la structure suivante :

(Z₁^{x-})ₘ(Z₂^{y+})ₙ

dans laquelle
Z₂ désigne un métal des colonnes 1 à 13 du tableau périodique des éléments, de préférence 1 ou 2, comme le sodium ou le potassium ;
Z₁^{x-} désigne un anion choisi parmi les ions CO₃²⁻, OH⁻, HCO₃²⁻, SiO₃²⁻, HPO₄²⁻, PO₄³⁻, B₄O₇²⁻, de préférence parmi les ions CO₃²⁻, OH⁻, SiO₃²⁻ ;
x désigne 1 , 2 ou 3 ;
y désigne 1, 2, 3 ou 4 ;
m et n désignent indépendamment l'un de l'autre 1, 2, 3 ou 4 ;
avec n.y=m.x.

De préférence, la base inorganique correspond à la formule suivante (Z₁^{x-} )ₘ(Z₂^{y+})ₙ, dans laquelle Z₂ désigne un métal des colonnes 1 et 2, du tableau périodique des éléments ; Z₁^{x-} désigne un anion choisi parmi les ions CO₃²⁻, OH⁻, SiO₃²⁻, x vaut 1, y désigne 1 ou 2, m et n désignent indépendamment l'un de l'autre 1 ou 2 avec n.y=m.x.

A titre de base inorganique utilisable selon l'invention on peut citer, le bicarbonate de sodium, le carbonate de potassium, la soude, la potasse, le métasilicate de sodium, le métasilicate de potassium.

On peut aussi utiliser en tant qu'agent alcalin des sels d'ammoniums.

Les sels d'ammonium utilisables dans la composition B selon la présente invention sont les sels d'ammonium (NH4+).

Les sels d'ammonium utilisables dans la composition B selon la présente invention sont de préférence choisi parmi les sels d'acide suivants : acétate, carbonate, bicarbonate, chlorure, citrate, nitrate, nitrite, phosphate, sulfate. De manière particulièrement préférée, le sel est le carbonate tel que le carbonate d'ammonium.

Selon un mode de réalisation particulier, la composition contient en tant qu'agents alcalins au moins une amine organique, de préférence au moins une alkanolamine. Lorsque la composition contient plusieurs agents alcalins dont une alkanolamine et de l'ammoniaque ou un de ses sels, la ou les amines organiques sont de préférence majoritaire en poids par rapport à la quantité ammoniac.

Généralement, l'émulsion (A) présente une teneur en agents alcalins allant de 0,1 à 40 % en poids, de préférence de 0,5 à 20 % en poids par rapport au poids de ladite composition.

L'émulsion (A) peut être préparée par des procédés de préparation classique d'émulsion directe mais aussi par un procédé par PIT. Le principe d'émulsification par inversion de phase en température (en Anglais : Phase Inversion Temperature ou PIT) est, dans son principe, bien connu de l'homme de l'art ; il a été décrit en 1968 par K. Shinoda (J. Chem. Soc. Jpn., 1968, 89, 435). Il a été montré que cette technique d'émulsification permet d'obtenir des émulsions fines stables (K. Shinoda et H. Saito, J. Colloïd Interface Sci., 1969,30, 258). Cette technologie a été appliquée en cosmétique dès 1972 par Mitsui et al. («Application of the phase-inversion-temperature method to the emulsification of cosmetics» ; T. Mitsui, Y. Machida and F. Harusawa, American. Cosmet. Perfum., 1972, 87, 33).

Le principe de cette technique est le suivant : on réalise le mélange d'une phase aqueuse et d'une phase huileuse, que l'on porte à une température supérieure à la température PIT, température d'inversion de phase du système, qui est la température à laquelle l'équilibre entre les propriétés hydrophile et lipophile du ou des émulsionnants mis en oeuvre est atteint ; à température élevée, c'est-à-dire supérieure à la température d'inversion de phase (>PIT), l'émulsion est de type eau-dans-huile, et, au cours de son refroidissement, cette émulsion s'inverse à la température d'inversion de phase, pour devenir une émulsion de type huile-dans-eau, et ceci en étant passée auparavant par un état de microémulsion. Selon ce mode de réalisation particulier, le tensio-actif non ionique présente un HLB compris entre 8 et 18. Il est de préférence choisi parmi les alcools gras éthoxylés, les acides gras éthoxylés, les glycérides partiels d'acides gras éthoxylés, les triglycérides d'acides gras polyglycérolés et leurs dérivés éthoxylés, et leurs mélanges. Par ailleurs, une telle émulsion présente une taille de particule inférieure à 4 microns, de préférence inférieure à 1µm.

De manière plus détaillée, on peut opérer de manière suivante pour obtenir une émulsion PIT :
1) Peser dans un récipient tous les constituants de l'émulsion directe (A)
2) Homogénéiser le mélange, par exemple au moyen d'un Rayneri 350 tours/min, et chauffer en augmentant progressivement la température au moyen d'un bain marie jusqu'à une température supérieure à la température d'inversion de phase T1, c'est-à-dire jusqu'à l'obtention d'une phase transparente ou translucide (zone de microémulsion ou de phase lamellaire) puis d'une phase plus visqueuse qui indique l'obtention de l'émulsion inverse (E/H).
3) Arrêter le chauffage et maintenir l'agitation jusqu'à retour à la température ambiante, en passant par la température d'inversion de phase T1, c'est-à-dire la température à laquelle se forme une émulsion H/E fine.
4) Lorsque la température est redescendue en dessous de la zone d'inversion de Phase en Température (T1), additionner les éventuels additifs et les matières premières thermosensibles.

On obtient une composition finale stable dont les gouttelettes de phase lipophile sont fines avec des tailles de 10 à 200 nm.

Dans la zone de formation d'une microémulsion (mélange translucide), les interactions hydrophiles et hydrophobes sont équilibrées car la tendance du tensioactif est de former aussi bien des micelles directes que des micelles inverses. Par chauffage au-delà de cette zone, il y a formation d'une émulsion E/H, car le tensioactif favorise la formation d'une émulsion eau dans huile. Puis lors du refroidissement en dessous de la zone d'inversion de phase, l'émulsion devient une émulsion directe (H/E).

L'émulsification par inversion de phase est expliquée en détail dans l'ouvrage T.Fôrster, W von Rybinski, A.Wadle, Influence of microemulsion phases on the préparation of fine disperse emulsions, Advances in Colloid and interface sciences, 58, 119-149, 1995 cité ici pour référence.

L'émulsion (A) peut également renfermer divers adjuvants utilisés classiquement dans les compositions pour l'éclaircissement des cheveux, tels que des polymères anioniques, cationiques, non ioniques, amphotères, zwitterioniques ou leurs mélanges ; des agents épaississants minéraux, et en particulier des charges telles que des argiles, le talc ; des agents épaississants organiques, avec en particulier les épaississants associatifs polymères anioniques, cationiques, non ioniques et amphotères ; des agents antioxydants ; des agents de pénétration ; des agents séquestrants ; des parfums ; des agents dispersants ; des agents filmogènes ; des agents conservateurs ; des agents opacifiants.

Elle peut éventuellement comprendre un ou plusieurs solvants organiques. A titre de solvant organique, on peut par exemple citer, les alcanols, linéaires ou ramifiés, en C₂-C₄, tels que l'éthanol et l'isopropanol ; le glycérol ; les polyols et éthers de polyols comme le 2-butoxyéthanol, le propylèneglycol, le dipropylèneglycol, le monométhyléther de propylèneglycol, le glycérol, le monoéthyléther et le monométhyléther du diéthylèneglycol, ainsi que les alcools aromatiques comme l'alcool benzylique ou le phénoxyéthanol, et leurs mélanges.

Le procédé est mis en oeuvre avec une composition (B) comprenant un ou plusieurs agents oxydants.

Plus particulièrement, le ou les agents oxydants sont choisis parmi le peroxyde d'hydrogène, le peroxyde d'urée, les bromates ou ferricyanures de métaux alcalins, les sels peroxygénés comme par exemple les persulfates, les perborates, les peracides et leurs précurseurs et les percarbonates de métaux alcalins ou alcalino-terreux.

Cet agent oxydant est avantageusement constitué par du peroxyde d'hydrogène et notamment en solution aqueuse (eau oxygénée) dont le titre peut varier, plus particulièrement, de 1 à 40 volumes (soit 0.3 à 12% de H2O2), et encore plus préférentiellement de 5 à 40 volumes (soit 1.5 à 12% de H2O2).

En fonction du degré d'éclaircissement souhaité, l'agent oxydant peut également comprendre un agent oxydant choisi de préférence parmi les sels peroxygénés.

La composition (B) est généralement une composition aqueuse. Par composition aqueuse, on entend une composition comprenant plus de 5 % en poids d'eau, de préférence plus de 10 % en poids d'eau, et de manière encore plus avantageuse plus de 20 % en poids d'eau

Cette composition (B) peut également comprendre un ou plusieurs solvants organiques tels que décrits précédemment. Elle peut aussi comprendre un ou plusieurs agents acidifiants.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Habituellement, le pH de la composition (B), est inférieur à 7.

Enfin, la composition (B) se présente sous diverses formes, comme par exemple une solution, une émulsion ou un gel.

Le procédé de l'invention peut être mis en oeuvre en appliquant l'émulsion (A) et la composition (B) successivement et sans rinçage intermédiaire.

Selon une autre variante, on applique sur les matières kératiniques, sèches ou humides, une composition obtenue par mélange extemporané, au moment de l'emploi, de l'émulsion (A) et de la composition (B). Selon ce mode de réalisation, le rapport pondéral des quantités de (A) / (B) et R2 varie de 0,1 à 10 de préférence de 0,2 à 2, mieux de 0,3 à 1.

En outre, indépendamment de la variante mise en oeuvre, le mélange présent sur les matières kératiniques (résultant soit du mélange extemporané de (A) et (B) ou de leur application successive partielle ou totale) est laissé en place pour une durée, en général, de l'ordre de 1 minute à 1 heure, de préférence de 5 minutes à 30 minutes.

La température durant le procédé est classiquement comprise entre la température ambiante (entre 15 à 25°C) et 80°C, de préférence entre la température ambiante et 60°C.

A l'issue du traitement, les matières kératiniques sont éventuellement rincées à l'eau, subissent éventuellement un lavage suivi d'un rinçage à l'eau, avant d'être séchées ou laissées à sécher.

Dans une variante préférée de l'invention les matières kératiniques sont des fibres kératiniques tels que les poils, cils et cheveux. Selon un mode de réalisatrion particulier de l'invention, la composition de coloration obtenue après mélange de l'émulsion (A) décrite précédemment et de la composition aqueuse (B) comprenant un agent oxydant est telle que après mélange la quantité de corps gras est supérieure à 20%, de préférence supérieure à 25 %, voire supérieure à 30 %.

Enfin, l'invention concerne un dispositif à plusieurs compartiments comprenant dans un premier compartiment une émulsion (A), et dans un second, une composition aqueuse (B) comprenant un ou plusieurs agents oxydants, ces compositions ayant été décrites auparavant.

### EXEMPLES

On prépare les compositions suivantes :

### Exemples de l'invention

L'émulsion A1 suivante a été préparée selon un procédé d'inversion de phase en température (procédé PIT) :

### Procédé de fabrication:

1- On chauffe la phase A au bain marie sous Rayneri (400 tr/min). On obtient une émulsion blanche fluide qui devient translucide vers 68°C (passage par une phase de microémulsion) et s'épaissit au-delà.
2- Dès que l'émulsion s'épaissit, on retire le bain marie : on laisse refroidir sous la même agitation.
3- Vers 50°C, on introduit le carbopol.
4- Au retour à la température ambiante, on introduit, l'éthanol, la monoéthanolamine et on réajuste l'eau perdue à l'évaporation (<5%).

On obtient ainsi une émulsion gélifiée translucide de taille de gouttes < 1 µm (viscosité = 72 UD M4 au rheomat, pH 11,5)

| **Emulsion A1** | | |
|---|---|---|
| **Phase** | **Nom** | **g%** |
| **A** | Beheneth-10 | 6,00 |
| | Glycérine | 9,00 |
| | Palmitate d'éthylhexyle | 17,70 |
| | Huile de vaseline | 45,00 |
| | Eau | 16,00 |
| **B** | Polymère carboxyvinylique synthétisé dans le mélange acétate d'éthyle/cyclohexane(JC :CARBOPOL 980) | 0,30 |
| **C** | Ethanol | 2,00 |
| | Monoéthanolamine | 4,00 |

Au moment de l'emploi, on mélange poids pour poids l'émulsion A1 à une composition aqueuse (B) oxydante comprenant une dispersion d'alcools gras (8%) dans l'eau et 12% d'eau oxygénée commercialisée sous le nom Platinium 20V.

Le mélange est ensuite appliqué sur une mèche de cheveu naturel châtain (hauteur de ton = 4). Le rapport de bain « mélange/mèche » est respectivement de 10/1 (g/g). Le temps de pose est de 30min à 27°C. A l'issue de ce temps, les mèches sont rincées, puis lavées avec du shampooing Elsève multivitamines. On obtient un bon niveau d'éclaircissement sans odeur.

L'émulsion A2 suivante a été préparée selon un procédé PIT.

### Procédé de fabrication:

- On chauffe la phase A au bain marie sous Rayneri (400 tr/min). On obtient une émulsion blanche fluide qui devient translucide vers 68°C (passage par une phase de microémulsion) et s'épaissit au-delà.
- Dès que l'émulsion s'épaissit, on retire le bain marie : On laisse refroidir sous la même agitation.
- Vers 50°C, on introduire le poloxamer.
- A Température ambiante, on introduit, l'éthanol, la monoéthanolamine, le bicarbonate de potassium préalablement dispersé dans 5g d'eau et on réajuste l'eau perdue à l'évaporation (<5%).

On obtient ainsi une émulsion gélifiée translucide de taille de gouttes < 1 µm (viscosité 8UD M4, taille des goutes < 1µm, pH =11.3)

| **Emulsion A2** | | |
|---|---|---|
| **Phase** | **Nom INCI** | **g%** |
| A | Beheneth-10 | 6,00 |
| | Sorbitol | 5,00 |
| | Huile de vaseline | 60,25 |
| | Eau | 10,00 |
| B | Ethanol | 2,00 |
| | Poloxamer 184 | 5,00 |
| | Bicarbonate de potassium | 1,75 |
| | Eau | 5,00 |
| | Monoéthanolamine | 5,00 |

Au moment de l'emploi, on mélange 1 poids l'émulsion A2 à 1,5 poids d'une composition aqueuse (B2) oxydante comprenant une dispersion d'alcools gras (8%) dans l'eau et 12% d'eau oxygénée : Platinium 20V .

Le mélange est ensuite appliqué sur une mèche de cheveu naturel châtain (hauteur de ton = 4). Le rapport de bain « mélange/mèche » est respectivement de 10/1 (g/g). Le temps de pose est de 30min à 27°C. A l'issue de ce temps, les mèches sont rincées, puis lavées avec du shampooing Elsève multivitamines.
On obtient un bon niveau d'éclaircissement sans odeur.

### Exemple comparatif

On a préparé la composition suivante à base d'ammoniaque :

| | g% |
|---|---|
| Alcool oléïque polyglycérolé à 2 moles de glycérol | 4 |
| Alcool oléïque polyglycérolé à 4 moles de glycérol | 5.69 MA |
| Acide oléïque | 3 |
| Amine oléïque à 2 moles d'oxyde d'éthylène vendue sous la dénomination commerciale ETHOMEEN 012 par la société Akzo | 7 |
| Laurylamino succinamate de diéthylaminopropyle, sel de sodium à 55% M.A. | 3.0 MA |
| Alcool oléïque | 5 |
| Diethanolamide d'acide oléïque | 12 |
| Alcool éthylique | 7 |
| Propylène glycol | 3.5 |
| Dipropylèneglycol | 0.5 |
| Monométhylether de propylèneglycol | 9 |
| Acétate d'ammonium | 0.8 |
| Ammoniaque à 20% de NH3 (41.15% de NH4OH) | 10 |
| Eau déminéralisée q.s.p. | 100 g |

Au moment de l'emploi, cette composition est mélangée poids pour poids avec de l'oxydant 20V (contenant ≈ 80% d'eau) comprenant une dispersion d'alcools gras (8%) dans l'eau et 12% d'eau oxygénée : Platinium 20V.. Le pH du mélange est de 9.9 ± 0.1

### Résultats

Les émulsions A1 et A2 de l'invention ne dégagent aucune odeur agressive, contrairement à la composition de l'exemple comparatif. De plus, et comme le montre le tableau ci-dessous, les niveaux d'éclaircissements obtenu avec les émulsions de l'invention ne sont pas significativement différents de celui obtenu avec l'exemple comparatif représentatif des compositions de l'état de la technique à base d'ammoniaque connues pour donner un bon niveau d'éclaircissement.

| | L* | a* | b* | ΔE |
|---|---|---|---|---|
| Cheveu non traité | 18,79 | 1,86 | 1,45 | / |
| Cheveu traité avec l'émulsion A1 | 21,42 | 5,72 | 6,22 | 6,68 |
| Cheveu traité avec l'émulsion A2 | 23,31 | 6,43 | 7,85 | 9,1 |
| Cheveu traité avec la composition comparative | 22,1 | 6,11 | 6,97 | 7,71 |

## Revendications

1. Procédé d'éclaircissement de fibres kératiniques dans lequel on met en oeuvre :
a) une émulsion directe (A) comprenant un ou plusieurs corps gras liquides à température ambiante et à pression atmosphérique, choisis parmi les alcanes de 6 à 16 atomes de carbone, les alcools gras, les esters d'acide gras, les esters d'alcool gras, les huiles minérales de plus de 16 atomes de carbone, les huiles non siliconées végétales, animales ou synthétiques et différents des acides gras, le ou les corps gras étant présent en quantité supérieure à 25 % en poids ; un ou plusieurs tensioactifs ; un ou plusieurs agents alcalins et une quantité d'eau supérieure à 5 % en poids, du pois total de l'émulsion,
b) une composition (B) comprenant un ou plusieurs agents oxydants, dans lequel on applique sur les fibres kératiniques, une composition obtenue par mélange extemporané, au moment de l'emploi, de l'émulsion (A) et de la composition (B), la composition obtenue après mélange de l'émulsion (A) et de la composition (B) est telle que la quantité de corps gras est supérieure à 20 %.

2. Procédé selon la revendication précédente, **caractérisé en ce que** l'émulsion (A) comprend plus de 50 % en poids de corps gras.

3. Procédé selon la revendication 1 ou 2 dans lequel la teneur en eau dans l'émulsion (A) est supérieure à 10 % en poids et de préférence comprise entre 10 et 50%.

4. Procédé selon l'une quelconque des revendications précédentes, **caractérisé en ce que** la teneur en corps gras est comprise entre 25 et 80% en poids par rapport au poids de l'émulsion (A).

5. Procédé selon l'une quelconque des revendications précédentes, **caractérisé en ce que** le ou les corps gras liquides ont un poids moléculaire supérieur ou égal à 360g/mol.

6. Procédé selon l'une quelconque des revendications précédentes, **caractérisé en ce que** l'émulsion (A) comprend un ou plusieurs tensioactifs non ioniques, plus particulièrement choisis parmi les tensioactifs non ioniques mono- ou poly-oxyalkylénés, mono- ou poly- glycérolés.

7. Procédé selon la revendication précédente **caractérisé par le fait que** le tensioactif de l'émulsion (A) est choisi parmi les produits d'addition d'oxyde d'éthylène avec l'alcool laurylique ; les produits d'addition d'oxyde d'éthylène avec l'alcool cetearylique, les produits d'addition d'oxyde d'éthylène avec l'alcool cetylique ; les produits d'addition d'oxyde d'éthylène avec l'alcool stearylique, les produits d'addition d'oxyde d'éthylène avec l'alcool isostearylique, les produits d'addition d'oxyde d'éthylène avec les acides laurique, palmitique, stearique ou behenique et leurs mélanges.

8. Procédé selon l'une quelconque des revendications précédentes, **caractérisé en ce que** l'émulsion (A) comprend un ou plusieurs agents alcalins.

9. Procédé selon l'une quelconque des revendications précédentes, **caractérisé en ce que** l'agent alcalin est choisi parmi les amines organiques, les bases inorganiques, les sels d'amines organiques et les sels d'ammoniums.

10. Procédé selon la revendication précédente, **caractérisé en ce que** l'amine organique est une alcanolamine, de préférence choisie parmi le 2-amino 2-méthyl 1-propanol, la monoéthanolamine ou leurs mélanges, un acide aminé basique choisi parmi l'arginine, l'histidine, la lysine, ou leurs mélanges.

11. Procédé selon l'une quelconque des revendications précédentes, **caractérisé en ce que** la composition (B) comprend un ou plusieurs agents oxydants choisis parmi le peroxyde d'hydrogène, le peroxyde d'urée, les bromates ou ferricyanures de métaux alcalins, les sels peroxygénés comme par exemple les persulfates, les perborates, les peracides et leurs précurseurs, et les percarbonates de métaux alcalins ou alcalino-terreux, de préférence le peroxyde d'hydrogène.

12. Procédé selon l'une quelconque des revendications précédentes dans lequel la composition (B) comprend plus de 5 % en poids d'eau, de préférence plus de 20 %.

13. Dispositif à plusieurs compartiments comprenant dans un premier compartiment, l'émulsion (A) selon l'une des revendications 1 à 11, dans un autre compartiment une composition (B) telle que définie à l'une des revendications 1, 11 à 12.

## Patentansprüche

1. Verfahren zur Aufhellung von Keratinfasern, wobei hergestellt werden:
a) eine direkte Emulsion (A), umfassend einen oder mehrere flüssige Fettkörper bei Umgebungstemperatur und Atmosphärendruck, ausgewählt aus Alkanen mit 6 bis 16 Kohlenstoffatomen, Fettalkoholen, Fettsäureestern, Fettalkoholestern, Mineralölen mit mehr als 16 Kohlenstoffatomen, pflanzlichen, tierischen oder synthetischen und von Fettsäuren verschiedenen silikonfreien Ölen, wobei der oder die Fettkörper in einer Menge von mehr als 25 Gew.-% vorhanden sind; einen oder mehrere grenzflächenaktive Stoffe; ein oder mehrere alkalische Mittel und eine Menge an Wasser von mehr als 5 Gew.-%, bezogen auf das Gesamtgewicht der Emulsion,
b) eine Zusammensetzung (B), umfassend ein oder mehrere Oxidationsmittel,
wobei auf die Keratinfasern eine Zusammensetzung aufgebracht wird, die durch spontanes Mischen, zum Zeitpunkt der Verwendung, der Emulsion (A) und der Zusammensetzung (B) erhalten wird, wobei die Zusammensetzung, die nach dem Mischen der Emulsion (A) und der Zusammensetzung (B) erhalten wird, derart ist, dass die Fettkörpermenge mehr als 20 % beträgt.

2. Verfahren nach dem vorhergehenden Anspruch,
**dadurch gekennzeichnet, dass** die Emulsion (A) mehr als 50 Gew.-% Fettkörper umfasst.

3. Verfahren nach Anspruch 1 oder 2, wobei der Wassergehalt in der Emulsion (A) größer ist als 10 Gew.-% und vorzugsweise zwischen 10 und 50 % liegt.

4. Verfahren nach einem der vorhergehenden Ansprüche,
**dadurch gekennzeichnet, dass** der Fettkörpergehalt zwischen 25 und 80 Gew.-%, bezogen auf das Gewicht der Emulsion (A), beträgt.

5. Verfahren nach einem der vorhergehenden Ansprüche,
**dadurch gekennzeichnet, dass** der oder die flüssigen Fettkörper ein Molekulargewicht größer oder gleich 360 g/mol aufweisen.

6. Verfahren nach einem der vorhergehenden Ansprüche,
**dadurch gekennzeichnet, dass** die Emulsion (A) einen oder mehrere nicht-ionische grenzflächenaktive Stoffe umfasst, mehr im Einzelnen ausgewählt aus nicht-ionischen grenzflächenaktiven Mono- oder Polyoxyalkylen-, Mono- oder Polyglycerin-Stoffen.

7. Verfahren nach dem vorhergehenden Anspruch,
**dadurch gekennzeichnet, dass** der grenzflächenaktive Stoff der Emulsion (A) ausgewählt wird aus Additionsprodukten von Ethylenoxid mit Laurylalkohol; Additionsprodukten von Ethylenoxid mit Cetearylalkohol, Additionsprodukten von Ethylenoxid mit Cetylalkohol; Additionsprodukten von Ethylenoxid mit Stearylalkohol, Additionsprodukten von Ethylenoxid mit Isostearylalkohol, Additionsprodukten von Ethylenoxid mit Laurin-, Palmitin-, Stearin- oder Behensäure und ihren Mischungen.

8. Verfahren nach einem der vorhergehenden Ansprüche,
**dadurch gekennzeichnet, dass** die Emulsion (A) ein oder mehrere alkalische Mittel umfasst.

9. Verfahren nach einem der vorhergehenden Ansprüche,
**dadurch gekennzeichnet, dass** das alkalische Mittel ausgewählt wird aus organischen Aminen, anorganischen Basen, Salzen organischer Amine und Ammoniumsalzen.

10. Verfahren nach dem vorhergehenden Anspruch,
**dadurch gekennzeichnet, dass** das organische Amin ein Alkanolamin ist, vorzugsweise ausgewählt aus 2-Amino-2-methyl-1-propanol, Monoethanolamin oder ihren Mischungen, einer basischen Aminosäure, ausgewählt aus Arginin, Histidin, Lysin oder ihren Mischungen.

11. Verfahren nach einem der vorhergehenden Ansprüche,
**dadurch gekennzeichnet, dass** die Zusammensetzung (B) ein oder mehrere Oxidationsmittel umfasst, ausgewählt aus Wasserstoffperoxid, Harnstoffperoxid, Bromaten oder Ferricyaniden von Alkalimetallen, peroxidhaltigen Salzen, wie beispielsweise Persulfaten, Perboraten, Persäuren und ihren Vorläufern, und Percarbonaten von Alkali- oder Erdalkalimetallen, vorzugsweise Wasserstoffperoxid.

12. Verfahren nach einem der vorhergehenden Ansprüche,
wobei die Zusammensetzung (B) mehr als 5 Gew.-% Wasser umfasst, vorzugsweise mehr als 20 %.

13. Vorrichtung mit mehreren Abteilen, umfassend in einem ersten Abteil die Emulsion (A) nach einem der Ansprüche 1 bis 11, in einem anderen Abteil eine Zusammensetzung (B), wie in einem der Ansprüche 1, 11 bis 12 definiert.

## Claims

1. Process for lightening keratin materials, in which the following are used:
a) a direct emulsion (A) comprising one or more fatty substances that are liquid at room temperature and at atmospheric pressure, chosen from alkanes of 6 to 16 carbon atoms, fatty alcohols, fatty acid esters, fatty alcohol esters, mineral oils of more than 16 carbon atoms, non-silicone plant, animal or synthetic oils, and other than fatty acids, the fatty substance(s) being present in an amount greater than 25% by weight, one or more surfactants; one or more alkaline agents and an amount of water greater than 5% by weight, of the total weight of the emulsion,
b) a composition (B) comprising one or more oxidizing agents, a composition obtained by extemporaneous mixing, at the time of use, of the emulsion (A) and of the composition (B) is applied to the keratin fibres, the composition obtained after mixing the emulsion (A) and the composition (B) is such that the amount of fatty substances is greater than 20%.

2. Process according to the preceding claim, **characterized in that** the emulsion (A) comprises more than 50% by weight of fatty substances.

3. Process according to Claim 1 or 2, in which the water content in the emulsion (A) is greater than 10% by weight and preferably between 10% and 50%.

4. Process according to any one of the preceding claims, **characterized in that** the fatty substance content is between 25% and 80% by weight relative to the weight of the emulsion (A).

5. Process according to any one of the preceding claims, **characterized in that** the liquid fatty substance(s) has (have) a molecular weight greater than or equal to 360 g/mol.

6. Process according to any one of the preceding claims, **characterized in that** the emulsion (A) comprises one or more nonionic surfactants more particularly chosen from monooxyalkylenated or polyoxyalkylenated, monoglycerolated or polyglycerolated nonionic surfactants.

7. Process according to the preceding claim, **characterized in that** the surfactant of the emulsion (A) is chosen from adducts of ethylene oxide with lauryl alcohol; adducts of ethylene oxide with cetearyl alcohol, adducts of ethylene oxide with cetyl alcohol; adducts of ethylene oxide with stearyl alcohol, adducts of ethylene oxide with isostearyl alcohol, adducts of ethylene oxide with lauric, palmitic, stearic or behenic acid, and mixtures thereof.

8. Process according to any one of the preceding claims, **characterized in that** the emulsion (A) comprises one or more alkaline agents.

9. Process according to any one of the preceding claims, **characterized in that** the alkaline agent is chosen from organic amines, mineral bases, organic amine salts and ammonium salts.

10. Process according to the preceding claim, **characterized in that** the organic amine is an alkanolamine, preferably chosen from 2-amino-2-methyl-1-propanol and monoethanolamine, or mixtures thereof, and a basic amino acid chosen from arginine, histidine and lysine, or mixtures thereof.

11. Process according to any one of the preceding claims, **characterized in that** the composition (B) comprises one or more oxidizing agents chosen from hydrogen peroxide, urea peroxide, alkali metal bromates or ferricyanides, and peroxygenated salts, for instance alkali metal or alkaline-earth metal persulfates, perborates and peracids and precursors thereof, and percarbonates, preferably hydrogen peroxide.

12. Process according to any one of the preceding claims, in which the composition (B) comprises more than 5% by weight of water, preferably more than 20%.

13. Multi-compartment device comprising, in a first compartment, the emulsion (A) according to one of Claims 1 to 11, in another compartment, a composition (B) as defined in one of Claims 1, 11 and 12.
